# EUROPEAN PATENT APPLICATION

(11) **EP 0 737 748 A1**
(43) Date of publication of application: **16.10.1996**
(21) Application number: 95103961.9
(22) Date of filing: 17.03.1995
(51) Int. Cl.: C12N 15/82

(54) **Efficient production of transgenic fertile homozygous plants from fresh microspores**

(71) Applicant: Hoechst NOR-AM AgrEvo Inc., Saskatoon, Saskatchewan S7N 3R3 (CA)
(72) Inventor: Dormann, Mathias, Dr., S7H 5L3 Saskatoon (CA); Wang, Hung-Mei, Dr., S7J 4Z5 Saskatoon (CA); Oelck, Michael, Dr., S7J 4J1 Saskatoon (CA)
(74) Representative: Schmidt, Werner, Dr.

(57) **Abstract**

The present invention relates to a modified transformation process and is intended to serve for improvement of transformation rates enabling the efficient production of transgenic fertile homozygous plants.

## Description

### 1. Introduction

Transgenic crop plants can be produced with the help of different transformation techniques. The method of choice for species and varieties, where plant regeneration from differentiated tissue is efficient, is Agrobacterium (Potrykus 1993). The Agrobacterium tumefaciens transformation system is simple and inexpensive. Plants are obtained with a limited number of gene insertions, and usually insertion of DNA occurs between two defined border sequences completely and exclusively (DeBlock 1993).

Microspores of higher plants have the potential to develop, under appropriate conditions directly into haploid or double haploid plants. Microspores of higher plants develop in vivo into pollen grains (gametophytic pathway). Microspore culture can induce an alternative (sporophytic) pathway which leads to the formation of haploid and doubled haploid embryos. These embryos develop directly into phenotypical normal plants. Misunderstandingly these embryos are sometimes referred to as pollen embryos. These homozygous lines can be tested immediately for their agronomic value as well as their the combining ability in the field (Huang 1992). Therefore microspores are theoretically the most suitable recipients for gene transfer in all species where it can be applied (Huang Bin & W. A. Keller 1989).

There were reports (Potrykus 1991, Heberle-Bors 1991, Langride et al. 1992) stating that it is difficult if not impossible to obtain transgenic plants by infection of pollen or proembryo with Agrobacterium." (Sangwan et al. 1993). More frequently multicellular haploid tissues such as microspore-derived embryos (Neuhaus et al. 1987, Swanson and Erickson 1989, Huang 1992) or haploid stem segments (Arnoldo 1992) have been used.

There have been three reports on the use of microspores for gene transfer in dicotyledon plants. Huang (1992) reported the use of microspores with the microinjection, particle bombardment and electroporation techniques and observed some transient gene expression, but no stable transformations. In over 50 experiments conducted only one plant was regenerated. Jardinaud et al. 1993 reported only transient gene expression after electroporation of B. napus microspores.

The only successful report on microspore transformation (Pechan 1989) claimed the production of transformed plants but the method presented has not been reproducible by others (Huang 1992).

### 2. Description of the invention

This invention relates to a modified transformation process and is intended to serve for improvement of transformation rates enabling the efficient production of transgenic fertile homozygous plants.

More particularly the present invention relates to a process for the transformation of plant cells comprising the following steps:
Ia. DNA Incubation or infection with Agrobacteria of freshly isolated plant cells.
Ib. Addition of cellolytic enzymes during DNA incubation or Agrobacterium cocultivation.
II. Wash with mucolytic enzymes after cocultivation with Agrobacterium.

More generally the invention relates to a process for improved transformation with the help of cellolytic enzymes and the use of mucolytic enzymes wherein said plant cells are microspores.

The term lytic enzyme used hereafter refers to any substance which dissolves cell wall components of the explant inducing a wound response or detergent effect which faciliates gene transfer, i. e. cellulase, hemicellulase, pectinase.

The term mucolytic enzyme used hereafter refers to any substance which dissolves prokaryotic cell wall components (mureines, peptidoglycanes), i. e. lysozyme.

The cellolytic enzymes applied during cocultivation are selected on basis of their capacity of a.) to increase the efficiency of transformation (detergent effect) and b.) to increase the yield of recoverable transformed plantlets. Besides improving transformation efficiency they help to overcome the detrimental effect of longer cocultivation with Agrobacteria (aggregation and overgrowth of explants) by dissolving selectively the fibrils produced by Agrobacterium during the coculture.

In a preferred embodiment the invention relates to a process wherein cellulases and lysozyme are used to enhance the transformation efficiency.

The solutions containing the lytic enzymes contain preferably from about 0,0004 to about 0,1 %, most preferably from about 0,004 to about 0,01 % of the cellulose and preferably from about 0,1 to about 10 %, especially prefered from about 0,5 to about 5 % and most preferably from about 0.75 to 1.5 % lysozyme.

The concentrations to be used depend on the length of incubation. The used cellulase concentration apply to an incubation period of 3 days. Accordingly, higher concentrations may be used if the incubation period is shorter.

The active enzymes used according the present invention are normally applied in form of compositions together with one or more acceptable supplements, and can be applied to the cells or explants to be treated, simultaneously or in succession, with further compounds.

These compounds can be antimicrotubule active compounds like colchicine or trifluralin that lead directly to an in vitro duplication of the haploid microspore genome, which additional effects transformation efficiency by synchronyzing the cell cycle of the microspore explants.

They can also be buffer substances or mixtures of several of these preparations, if desired together with further medium supplements customaily employed in the art of transformation.

Suitable media can be semisolid or liquid and correspond to the substances ordinarily employed in transformation technology, e. g. B₅ medium (GAMBORG et al. 1968).

The number of applications and the rate of application depend on the Agrobacterium strain and its culture, the time and temperature during cocultivation and the microspore material, i. e. the species.

Another object of the invention is to provide a new process for transforming plant explants and plant cells which comprises at least one additional washing step with mucolytic enzymes after cocultivation with Agrobacterium. The washing procedure may be performed preferrably as described in material and methods. Said washing is repeated preferably one to two times.

The solution which may be used to wash the plant cells may be especially in the form of Tris HCL (pH 8.0) as described in Sambrook, Fritsch & Maniatis 1989).

The DNA construct can be introduced into the plant cell using different Agrobacteria transformation techniques that are described in the art. These methods include Agrobacterium mediated transformation as described by Hoekema and An et al.

A preferred method of introducing the nucleic acid segments into plant cells is to infect microspores with Agrobacterium tumefaciens carrying an inserted DNA construct. Under appropriate conditions known in the art, the transformed plant cells are grown to form shoots, roots, and develop further into plants. The nucleic acid segments or constructs can be introduced into appropriate plant cells, for example, by means of the Ti plasmid of Agrobacterium tumefaciens. The Ti plasmid is transmitted to plant cells upon infection by Agrobacterium tumefaciens, and is stably integrated into the plant genome.

The transformation of plants in accordance with the invention may be carried out in essentially any of the various ways known to those skilled in the art of plant molecular biology. See, in general, Methods in Enzymology Vol. 153 ("Recombinant DNA Part D") 1987, Wu and Grossman Eds., Academic Press, incorporated herein by reference. As used herein, the term transformation means alteration of the genotype of a host plant by the introduction of a nucleic acid sequence.

The agrobacterium strains customarily employed in the art of transformation are described, for example, in White (1993) or Gruber and Crosby (1993).

Ti plasmids contain two regions essential for the production of transformed cells. One of these, named transfer DNA (T DNA), induces tumor formation. The other, termed virulent region, is essential for the introduction of the T DNA into plants. The transfer DNA region, which transfers to the plant genome, can be increased in size by the insertion of the foreign nucleic acid sequence without its transferring ability being affected. By removing the tumor-causing genes so that they no longer interfere, the modified Ti plasmid can then be used as a vector for the transfer of the gene constructs of the inventioninto an appropriate plant cell, such being a "disabled Ti vector". In the binary system, to have infection, two plasmids are needed: a T-DNA containing plasmid and a vir plasmid. Any one of a number of T-DNA containing plasmids can be used, the only requirement is that one be able to select independently for each of the two plasmids.

After transformation of the plant cell or plant, those plant cells or plants transformed by the Ti plasmid so that the desired DNA segment is integrated can be selected by an appropriate phenotypic marker. These phenotypic markers include, but are not limited to, antibiotic resistance, herbicide resistance or visual observation. Other phenotypic markers are known in the art and may be used in this invention.

The DNA constructs used in instant invention consist of a transcription initiation region and, under the control of the transcription initiation region, a DNA sequence to be transcribed. The DNA sequence may comprise a natural open reading frame including transcribed 5' and 3' flanking sequences. Alternatively, it may comprise an anti-sense sequence that encodes the complement of an RNA molecule or portion thereof.

The initiation regions may be used in a variety of contexts and in combination with a variety of sequences. The RNA coded sequences of a gene may be those of a natural gene, including the open reading frame for protein coding and frequently the 5' and 3' untranslated sequences. The RNA translational initiation sequences are included in the constructs, either from the promoter domain or from the attached coding sequences.

Attached to the above sequences are approriate transcription termination and polyadenylation sequences.

Examples of the above sequences to be expressed from the constructs of the subject invention include: antisense RNAs (for gene suppression): nutritionally important proteins: growth promoting factors: proteins giving protection to the plant under certain environmental conditions, e. g. proteins giving resistance to metal or other toxicity: stress related proteins giving tolerance to extremes of temperature, freezing, etc. proteins of specific commercial value; increased level of proteins, e. g., enzymes of metabolic pathways, increased levels of products of structural value to a plant host, e. g., herbicide resistance.

The subject constructs will be prepared employing cloning vectors, where the sequences may be naturally occuring, mutated sequences, synthetic sequences, or combinations thereof. The cloning vectors are well known and comprise prokaryotic replication systems, markers for selection of transformed host cells, and restriction sites for insertion or substitution of sequences. For transcription and optimal expression, the DNA may be transformed into a host, e. g. plant cells, preferably microspores for integration into the genome, where the subject construct is joined to a marker for selection or is co-transformed with DNA encoding a marker for selection.

The selection of plant cells which have been transformed is enabled by the use of a selectable marker gene which is also transferred. The expression of the marker gene confers a phenotypic trait that enables the selection. Examples for such genes are those coding for antibiotica or herbicide resistance, e.g. neomycin or phosphinothricin resistance, or the glucorinidase gene.

As used herein, "plant" refers to either a whole plant, a plant part, a plant cell, or a group of plant cells. The class of plants which can be used in the method of the invention is generally as broad as the class of higher plants amenable to transformation techniques, including both monocotyledonous and dicotyledonous plants. It is known that practically all plants can be regenerated from cultured cells or tissues, including but not limited to all major cereal crop species, sugarcane, sugar beet, cotton, fruit and other trees, legumes and vegetables.

Limited knowledge presently exists on whether all of these plants can be transformed by Agrobacterium. Species which are a natural plant host for Agrobacterium may be transformable in vitro. Although monocotyledonous plants, and in particular cereals and grasses, are not natural hosts to Agrobacterium, work to transform them using Agrobacterium has. All plants from which microspores can be isolated and cultured to give whole regereated plants can be transformed by the present invention so that whole plants are recovered which contain the transferred foreign gene.

Some suitable plants include, for example, species from the genera Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Hyoscyamus, Lycopersicon, Nicotiana, Solanum, Petunia, Digitalis, Majorana, Ciohorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia, Glycine, Lolium, Zea, Triticum, Sorghum, and Dutura.

Examples of families that are of special interest are Solanaceae and Brassicaceae. Examples of species of commercial interest that can be protected include:

| | |
|---|---|
| - tobacco, Nicotiana tabacum L. | - tomato, Lycopersicon esculentum Mill, |
| - potato, Solanum tuberosum L., | - petunia, Petunia hybrida (Solanaceae) |
| - Canola/Rapeseed, | - Brassica napus L., |
| - cabbage, broccoli, kale etc., | - Brassica oleracea L., |
| - mustards Brassica juncea L., | - Brassica nigra L., |
| - Sinapis alba L. (Brassicaceae), | |
| - sugar beet, Beta vulgaris,(Chenopodiaceae), | |
| - cucumber, Curcurbita sp. (Curcurbitaceae), | |
| - cotton, Gossypium sp., (Malvaceae), | |
| - sunflower, Helianthus annuus, | |
| - lettuce Lactuca sativa, (Asteraceae = Compositae), | |
| - pea, Pisum sativum, | |
| - soybean, Glycine max and alfalfa, Medicago sp. (Fabaceae = Leguminoseae), | |
| - asparagus, Asparagus officinalis; | - gladiolus, Gladiolus sp., (Lilaceae); |
| - corn, Zea mays and | - rice, Oryza sativa (Poaceae). |

In an preferred embodiment the process is used to transform plants such as members of the species brassica as for example B. napus, B. rapa, B. juncea, B. oleracea, B. carinata and others.

The invention additionally relates to brassica plants which have been regenerated out of cells which have been transformed according to instant invention.

Also non Brassica species such as corn, barley, wheat, and rice will be transformable with this method.

By taking advantage of genetic engineering the gene responsible for the production of novel traits can be transferred to different kinds of plant cells, which can be regenerated to whole plants as for example immature zygotic embryos, protoplasts or microspores, which (after microspore culture) develop directly into (sporophytic) embryos/plants.

The present invention is directed also to the use of microspores for a new haploid transformation system. Microspores refers to freshly isolated microspores up to 48 hours which comprise mainly of single cells as the first cell division is usually visible three days after culture. The microspore system offers advantages over other explant methods in that regeneration leads to the production of haploid plants or homozygous diploid plants. This offers simplicity for genetic studies and one-step fixation of genes for breeding purposes.

Another object of the invention is to provide a new process for transforming plant explants and plant cells by using not only Agrobacteria but also Polyethylen. The direct gene transfer method (via polyethylene glycol) is of special interest with regard to the embryogenic Brassica microspore culture systems. Up to know PEG mediated direct gene transfer has been demonstrated only by transient gene expression in maize as embryo production is not yet possible with the maize system (Fennell and Hauptmann 1992).

The transformation and regeneration can be carried out as described in the materials and methods and in the examples.

In general, preparation of plasmid DNA, restriction enzyme digestion, agarose gel electrophoresis of DNA, Southern blots, DNA ligation and bacterial transformation were carried out using standard methods. (Maniatis et al., Molecular Cloning, a Laboratory Manual, Cold Spring Harbor Laboratory (1982), referred to herein as "Maniatis" and hereby incorporated by reference.)

### 3. Materials and Methods

The following disclosure provides the technique how the plant cells can be transformed. The disclosure will then be completed with the description of the conditions under which the cells can be transformed, also merely by way of examples for non-limitative illustration purposes.

Freshly isolated microspores or microspores cultured up to 48 h (10⁵/ml NLN containing 17 % sucrose) were mixed with Agrobacterium tumefaciens cells (diluted 10⁻⁵ to 10⁻⁷ times from a 0.9 O. D. spectrophotometric standard). The cultures were supplemented with cellolytic enzymes and incubated for 72 hours following the temperature regime described by Baillie et al. (1992).

Subsequently (after three days of cocultivation), the cultures were washed gently by discarding the supernatant and adding fresh NLN medium containing 10% sucrose sucrose with 500 mg/l carbenicillin (or 300 mg/l Timentin). As the microspores adhere tightly to the bottom of the petri plate at this stage of infection, there is very little loss of microspores.

The microspores adhering to the bottom of the petri plates are then gently scraped and loosened from the bottom of the plate using soft rubber policemen (Fisher Scientific). Then the fresh medium and microspores were transferred with 10 ml pipette into 50 ml Falcon tubes for centrifugation.

Centrifugation was carried out two times for three minutes (at 200 g). Microspores were suspended and centrifugeg at least once in lysozyme and 10 mM Tris HCL buffer pH 8.0. Finally fresh NLN medium plus 10 % sucrose and 500 mg/l carbenicillin (or 300 mg/l Timentin) was added and the cultures were incubated according to Baillie et al. 1992.

The cultures are kept at 24 °C until they are four weeks old as the developing embryos are usually a week slower than non-infected control embryos. The timeframe from microspore isolation until transformed plantlet in the greenhouse takes 4 months.

The efficiency of transformation was very satisfactory (about 15 percent).

The transformation method using Agro. is relatively simple, highly reproducible and has a high possibility of being extrapolated to other embryogenic Brassica microspore culture systems. In all species where microspores can be regenerated or another culture systems exist, this method can be expected to generate transgenic haploid plants in a one step procedure.

### 4. Experimental Data

### 4.1. Optimization of cocultivation conditions for improved transformation efficiency and embryo recovery

- Material:: Brassica rapa, CV2
- DNA construct:: glufosinate (Phosphinothricin, L-PPT) resistance gene under control of 35S promotor
- Preculture:: Incubator 4 (10/5°C, 16 h photoperiod)

| Treatment | 0 (control) | 1 | 2 | 3 | 4 |
|---|---|---|---|---|---|
| day 0 | Initiation of microspore culture | Infection with cellulase | Infection without cellulase | Infection with cellulase | Infection without cellulase |
| day 2 | Transfer | Transfer | Transfer | Transfer | Transfer |
| day 3 | Centrifuge Handwash | Centrifuge Handwash | Centrifuge Handwash | Centrifuge Handwash | Centrifuge Handwash |
| day 23 | count | count | count | count | count |
| day 30 | greening | greening | greening | greening | greening |
| day 39 | plating | plating | plating | plating | plating |
| day 54 | rooting | rooting | rooting | rooting | rooting |
| day 59 | 0 | 10 | 4 | 11 | 6 |
| 0: Control 1: + cellulase + pRD 320 (10⁻⁶) 2: - cellulase + pRD 320 (10⁻⁶) 3: + cellulase + pRD 320 (10⁻⁷) 4: - cellulase + pRD 320 (10⁻⁷) day 0: Initiation of microspore culture after Baillie (1992) but without amino acids. Incubation for two days at 32°C. day 2: Transfer of plates to 24°C incubator. day 3: Media change to NLN10/Centrifuge wash was performed 2 times with 1600 rpm. day 23: Embryo count: 400-500 embryos plate. day 30: Greening up of embryos under continous light at 22°C on shaker at 75 rpm. day 39: Plating on OB5 solid medium. day 54: Start L-PPT Rooting Assay (20 mg/l L-PPT). day 59: Transfer of putative transformants to OB5 solid medium. | | | | | |

The data for the recovered embryos and putative transformants are summarized in the attached graphics. The results show that the addition of cellulase does not only dissolve the fibrils of the Agrobacteria to enable centrifuge wash, but also increased transformation efficiency, obviously by dissolving cellulose of the plant material. Cellulase on the other hand reduced the number of embryos recovered slightly within each level of the tested Agrobacterium concentration. The number of recovered embryos and consequently also of the transformants was always higher in the group of embryos which wee cocultivated with the lower (10⁻⁷) Agrobacterium concentration.

### 4.2. Compare of the effect of different washing procedures on embryo yield

- Material:: Brassica rapa, CV2
- DNA construct:: see 4.1
- Preculture:: Incubator 4 810/5°C, 16 h photoperiod), flower buds, stored at 4°C for two days prior to MSC.

| | 0 (Control) | 1 | 2 | 3 | 4 |
|---|---|---|---|---|---|
| day 0 | Initiation | Initiation | Initiation | Initiation | Initiation |
| day 3 | transfer | transfer | transfer | transfer | transfer |
| day 5 | Media change | Media change | Media change | Media change - cellulase | Media change + cellulase |
| day 6 | Media change | Media change + lysozyme treatment | Handwash (lots of connected fibrils) | Centrifuge wash (fibrils dissolved) | Centrifuge wash + lysozyme (fibrils dissolved) |
| day 22 | 484 embryos/plate | 390 embryos/plate | no embryos | 27 embryos/plate | 58 embryos/plate |
| 0: uninfected control, 1: uninfected control & the application of lysozyme during media change, 2: hand wash, 3: cellulase + centrifuge Wash, 4: as 3 but with additional lysozyme treatment at Wash I. day 0: Initiation of microspore culture after Baillie (1992) but without amino acids. Incubation for two days at 32°C. day 3: Transfer of plates to 24°C incubator. day 5: Media change to NLN10/Centrifuge wash was performed 2 times with 1600 rpm. day 6: Lysozyme treatment: 1 ml of a 500 mg/10 ml stock for 50 ml culture. day 22: Lysozyme treatment doubled the number of recovered embryos. Mean number of embryos of 4 differs significantly from 2 (tested with 8 replicates per treatment). | | | | | |

### 5. References

Arnoldo, M. 1992. Development of an efficient Agrobacterium- mediated transformation system in Brassica napus. Canada: University of Toronto; Thesis

Baillie, A. M. R., Epp, D. J., Hutcheson, D., Keller, W. A. 1992. In vitro culture of isolated microspores and regeneration of plants in Brassica campestris Plant Cell Rep 11 : 234 - 237.

Creissen, G., Smith, C., Francis, R., Reynolds, H., Mullineaux, P. 1990. Agrobacterium- and microprojectile-mediated viral DNA delivery into barley microspore-derived cultures. Plant Cell Rep 8 : 680 - 683.

Fennell, A., Hauptmann, R. 1992. Electroporation and PEG delivery of DNA into maize microspores. Plant Cell Rep 11 : 567 - 570.

Huang, B. 1992. Genetic manipulation of microspores and microspore-derived embryos. In Vitro Cell. Dev. Biol. 28P : 53 - 58. (Review)

Jardinaud, M-F., Souvré, A., Alibert, G. 1993. Transient GUS gene expression in Brassica napus electroporated microspores. Plant Sci 93 : 177 - 184.

Neuhaus, G., Spangenberg, G., Mittelsten Scheid, O., Schweiger, H-G. 1987. Transgenic rapeseed plants obtained by the microinjection of DNA into microspore-derived embryoids. Theor Appl. Genet 75 : 30 - 36.

Pechan, P. M. 1989. Successful cocultivation of Brassica napus microspores and proembryos with Agrobacterium. Plant Cell Rep 8 : 387 - 390.

Swanson, E. B., Erickson, L. R. 1989. Haploid transformation in Brassica napus using an octopine-producing strain of Agrobacterium tumefaciens. Theor. Appl. Genet. 78 : 831 - 835.

## Claims

1. Process for the transformation of plant cells comprising the following steps:
Ia. DNA Incubation or infection with Agrobacteria of freshly isolated plant cells.
Ib. Addition of cellolytic enzymes during DNA incubation or Agrobacterium cocultivation.
II. Wash with mucolytic enzymes after cocultivation with Agrobacterium.

2. Process according to claim 1 in which the plant cells is derived from Brassicaeae.

3. Process according to claim 1 in which the lytic enzyme is a cellulase and/or lysozyme.

4. Use of lytic enzymes which kill the agrobacteria to increase the number of surviving transformants.
